# EUROPÄISCHE PATENTSCHRIFT

(11) **EP 2 818 093 B1**
(45) Veröffentlichungstag und Bekanntmachung des Hinweises auf die Patenterteilung: **14.11.2018**
(21) Anmeldenummer: 14166329.4
(22) Anmeldetag: 29.04.2014
(51) Int. Cl.: A61B 1/00, G02B 27/00, A61B 1/04, G02B 13/14, G02B 27/14, G02B 23/24

(54) **Endoskop**
Endoscope
Endoscope

(30) Priorität: 25.06.2013 DE 102013212111
(43) Veröffentlichungstag der Anmeldung: 31.12.2014
(73) Patentinhaber: Henke-Sass, Wolf GmbH, 78532 Tuttlingen (DE)
(72) Erfinder: Rehe, Oliver, 78573 Wurmlingen (DE)
(74) Vertreter: Patentanwälte Geyer, Fehners & Partner mbB

(56) Entgegenhaltungen:
- US-A1- 2011 249 323
- US-A1- 2012 002 956

## Beschreibung

Die vorliegende Erfindung betrifft ein Endoskop, das für eine Abbildung sowohl im sichtbaren Spektrum als auch im nahen Infrarot geeignet ist.

Wenn sowohl eine Abbildung im sichtbaren Spektrum als auch im nahen Infrarot durchgeführt werden soll, besteht häufig die Schwierigkeit, daß bekannte Endoskope (insbesondere ihre Abbildungsoptik) dafür nicht ausgelegt sind. Dies führt dann dazu, daß die Fokusebene für Licht aus dem sichtbaren Spektrum nicht mit der Fokusebene für Licht aus dem nahen Infrarot zusammenfällt. Dieser in axialer Richtung vorliegende Abstand der beiden Fokusebenen wird häufig auch als chromatischer Längsfehler bezeichnet.

Aus der US 2011/0249323 A1 ist es bekannt, zwei Dove-Prismen an ihren längsten Seiten zusammenzufügen und dazwischen eine dichroitische Teilerschicht vorzusehen, um so Licht aus dem sichtbaren Spektrum im wesentlichen in einem der beiden Dove-Prismen und Licht aus dem nahen Infrarot im wesentlichen in dem anderen der beiden Dove-Prismen zu führen und danach wieder zu überlagern (Fig. 3 der US 2011/0249323 A1). Durch die Wahl der Prismenmaterialien können so die optischen Weglängen unterschiedlich eingestellt werden. Nachteilig ist hier jedoch, daß einerseits ein lateraler Versatz zwischen dem in die Prismenanordnung einfallenden Lichtstrahl und dem aus der Prismenanordnung ausfallenden Lichtstrahl vorliegt. Des weiteren erfolgt die Kompensation des chromatischen Längsfehlers rein über die unterschiedlichen Materialien, da die geometrischen Wege für Licht aus dem sichtbaren Spektrum und Licht aus dem nahen Infrarot gleich sind, so daß die Möglichkeit der Kompensation des chromatischen Längsfehlers stark eingeschränkt ist.

Die US 2012/0002956 A1 zeigt ein Endoskop mit den Merkmalen des Oberbegriffs des Anspruchs 1.

Ausgehend hiervon ist es daher Aufgabe der Erfindung, ein Endoskop bereitzustellen, bei dem eine gute Korrektur des chromatischen Längsfehlers möglich ist.

Die Aufgabe wird durch ein Endoskop mit einem Hauptteil, einem mit dem Hauptteil verbundenen Endoskopschaft und einer innerhalb des Endoskopschaftes und des Hauptteiles angeordneten Abbildungsoptik, die ein vor dem dem Hauptteil abgewandten Ende des Endoskopschaftes befindliches Objekt in eine Fokusebene der Abbildungsoptik abbildet, gelöst, wobei das Hauptteil an dem dem Endoskopschaft abgewandten Ende einen Kameraanschluss aufweist, an dem eine Kamera angebracht werden kann, wobei die Abbildungsoptik ein im Hauptteil angeordnetes Korrekturmodul aufweist, durch das Licht zur Abbildung des Objektes geführt ist und das eine Teilereinheit und eine Überlagerungseinheit umfaßt sowie frei von abbildenden optischen Elementen ist, wobei das Licht dem Korrekturmodul über einen ersten Strahlengang zugeführt ist, der mittels der Teilereinheit in einen ersten und in einen zweiten Teilstrahlengang aufgeteilt ist, wobei die Teilereinheit Licht aus dem sichtbaren Spektrum in einen der beiden Teilstrahlengänge und Licht aus dem nahen Infrarot in den anderen der beiden Teilstrahlengänge führt, und die Überlagerungseinheit das Licht aus beiden Teilstrahlengängen überlagert und in einen zweiten Strahlengang führt, der koaxial zum ersten Strahlengang verläuft, wobei die optischen Weglängen der beiden Teilstrahlengänge für das Licht aus dem sichtbaren Spektrum und das Licht aus dem nahen Infrarot so verschieden gewählt sind, daß der chromatische Längsfehler im Bereich der Fokusebene zwischen dem Licht aus dem sichtbaren Spektrum und dem Licht aus dem nahen Infrarot kompensiert ist.

Durch die koaxiale Anordnung der beiden Strahlengänge wird in vorteilhafter Weise erreicht, daß die laterale Ausdehnung des Korrekturmoduls minimiert werden kann. Ferner kann die Differenz der optischen Weglängen unabhängig von der lateralen Ausdehnung über die Längen der beiden Teilstrahlengänge und der in diesen verwendeten Materialien frei eingestellt werden.

Die Kompensation bzw. Korrektur des chromatischen Längsfehlers kann teilweise oder vollständig sein. Auf jeden Fall wird der chromatische Längsfehler verringert.

Da das Korrekturmodul bei dem erfindungsgemäßen Endoskop frei von abbildenden optischen Elementen ist, weist es somit keine gekrümmte und somit optisch wirksame Grenzfläche auf. Alle vorhandenen Grenzflächen sind plan ausgebildet.

Das Korrekturmodul ist daher bevorzugt als afokales Korrekturmodul ausgebildet.

Somit ist das Korrekturmodul an sich ohne großen Aufwand und somit auch kostengünstig herstellbar.

Insbesondere kann der erste Teilstrahlengang koaxial zu den beiden Strahlengängen sein. Damit ist es möglich, daß die laterale Ausdehnung (also quer zur Längsrichtung der beiden Strahlengänge) des Korrekturmoduls möglichst gering ist.

Ferner kann der zweite Teilstrahlengang einen Abschnitt aufweisen, der parallel zu beiden Strahlengängen ist. Dies vereinfacht die Herstellung des Korrekturmoduls, da ein solcher Abschnitt des zweiten Teilstrahlengangs durch nur rechtwinklige Strahlengangfaltungen erreichbar ist.

Bei dem erfindungsgemäßen Endoskop kann der Teilstrahlengang, in dem das Licht aus dem nahen Infrarot geführt ist, einen sich geradlinig erstreckenden Block mit einer ersten Brechzahl, und der Teilstrahlengang, in dem das Licht aus dem sichtbaren Spektrum geführt ist, ein Medium (z.B. Luft) mit einer zweiten Brechzahl aufweisen, wobei die erste Brechzahl für das Licht aus dem nahen Infrarot größer ist als die zweite Brechzahl für das Licht aus dem sichtbaren Spektrum.

Das Medium kann z.B. gasförmig und der geradlinig erstreckende Block kann z.B. als Festkörper (beispielsweise aus Glas oder Kunststoff) ausgebildet sein. Es ist ferner möglich, daß das Medium als Festkörper (z.B. Glas oder Kunststoff) ausgebildet ist.

Insbesondere kann der erste Teilstrahlengang geradlinig und der zweite Teilstrahlengang U-förmig sein.

Bei dem erfindungsgemäßen Endoskop kann die Teilereinheit und die Überlagerungseinheit jeweils als Teilerwürfel ausgebildet sein.

Ferner kann der zweite Teilstrahlengang zwei Umlenkprismen aufweisen.

Insbesondere kann jedes Umlenkprisma mit jeweils einem Teilerwürfel in mechanischem Kontakt stehen.

Der zweite Teilstrahlengang kann einen geradlinigen Block zwischen beiden Umlenkprismen aufweisen. Der geradlinige Block kann im mechanischen Kontakt mit den beiden Umlenkprismen stehen.

Ferner kann zusätzlich oder alternativ der erste Teilstrahlengang einen sich geradlinig erstreckenden Block aufweisen, der zwischen beiden Teilerwürfeln angeordnet ist. Insbesondere kann der Block in mechanischem Kontakt mit beiden Teilerwürfeln stehen.

Das erfindungsgemäße Endoskop kann als medizinisches oder technisches Endoskop ausgebildet sein. Ferner kann das erfindungsgemäße Endoskop hermetisch dicht oder autoklavierbar sein.

Der Endoskopschaft kann als starrer Endoskopschaft, als Endoskopschaft mit einem abwinkelbaren distalen Ende oder als flexibler Endoskopschaft ausgebildet sein.

Am Kameraanschluss kann eine Kamera angebracht werden, mit der das mittels der Abbildungsoptik abgebildete Objekt aufgenommen werden kann. Die Kamera kann so ausgebildet sein, daß sie sowohl ein Bild mit Licht aus dem sichtbaren Spektrum als auch ein Bild mit Licht aus dem nahen Infrarot (gleichzeitig und/oder zeitlich nacheinander) aufnehmen kann.

Insbesondere wird ein System aus dem erfindungsgemäßen Endoskop und einer damit verbundenen Kamera bereitgestellt.

Unter Licht aus dem sichtbaren Spektrum wird hier insbesondere Licht mit einer Wellenlänge aus dem Bereich von 380 bis 750 nm und insbesondere 400 bis 700 nm verstanden. Unter Licht aus dem nahen Infrarot wird hier insbesondere Licht mit einer Wellenlänge aus dem Bereich von 700 nm bis 3 µm, 780 nm bis 3 µm, 700 bis 1500 nm und insbesondere 780 bis 1500 nm verstanden. Auf jeden Fall überlappen sich die Wellenlängenbereiche für Licht aus dem sichtbaren Spektrum und Licht aus dem nahen Infrarot nicht.

Die Fokusebene der Abbildungsoptik kann im Hauptteil oder hinter dem Hauptteil und somit außerhalb des Hauptteils liegen.

Der erste Strahlengang endet bevorzugt an der Teilereinheit des Korrekturmoduls. Der zweite Strahlengang beginnt bevorzugt an der Überlagerungseinheit des Korrekturmoduls. Die beiden Teilstrahlengänge laufen bevorzugt jeweils von der Teilereinheit bis zur Überlagerungseinheit.

Das erfindungsgemäße Endoskop kann weitere, dem Fachmann bekannte Elemente aufweisen, die zum Betrieb des erfindungsgemäßen Endoskopes notwendig sind.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
Fig. 1 eine schematische Ansicht einer Ausführungsform des erfindungsgemäßen Endoskops,
Fig. 2 eine vergrößerte Ansicht des Korrekturmoduls der Abbildungsoptik, und
Fig. 3 eine Abwandlung des Korrekturmoduls gemäß Fig. 2.

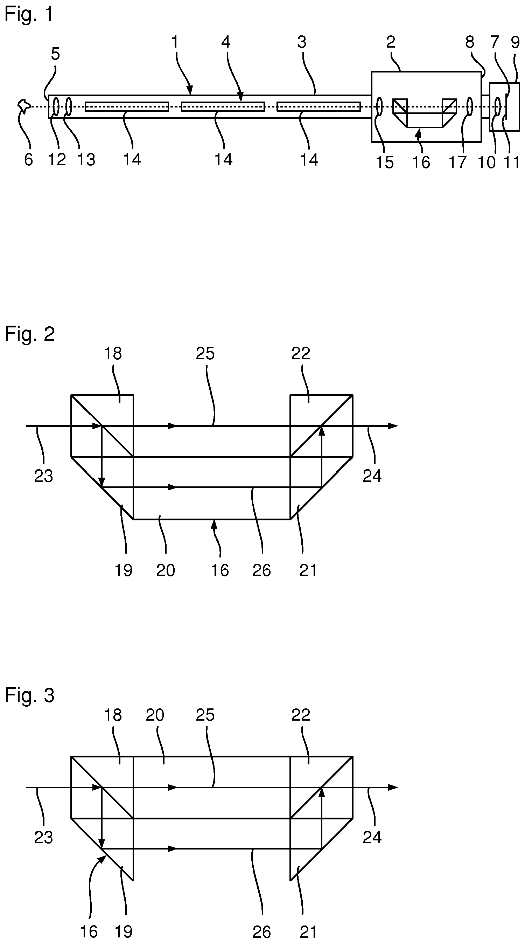

Bei der in Fig. 1 gezeigten Ausführungsform weist das erfindungsgemäße Endoskop 1 einen Hauptteil 2 und einen mit dem Hauptteil 2 verbundenen Endoskopschaft 3 auf.

Ferner umfaßt das Endoskop 1 eine Abbildungsoptik 4, die ein vor dem dem Hauptteil 2 abgewandten distalen Ende 5 des Endoskopschaftes 3 befindliches Objekt 6 in eine Fokusebene 7 der Abbildungsoptik 4 abbildet. Wie in Fig. 1 schematisch dargestellt ist, kann an dem dem Endoskopschaft 3 abgewandten proximalen Ende 8 des Hauptteils 2 eine Kamera 9 (beispielsweise lösbar) befestigt sein, die eine Kameraoptik 10 und einen dieser nachgeordneten Aufnahmesensor 11 aufweist. Die Abbildungsoptik 4 ist bevorzugt so ausgelegt, daß die Fokusebene 7 mit der Position des Aufnahmesensors 11 zusammenfällt, so daß eine scharfe Aufnahme des Objektes 6 durchgeführt werden kann.

Die Kameraoptik 10 kann als separater Koppler bzw. Coupler ausgebildet sein, der am proximalen Ende 8 des Hauptteils 2 z.B. lösbar befestigt werden kann. An dem dem Hauptteil 2 abgewandten Ende des Kopplers kann dann ein Kameramodul mit einem entsprechenden Aufnahmesensor 11 wiederum bevorzugt lösbar befestigt werden. Der Koppler kann beispielsweise eine Fokussierfunktion bereitstellen.

Es ist auch möglich, das erfindungsgemäße Endoskop 1 so abzuwandeln, daß der Koppler fest mit dem Hauptteil 2 verbunden ist. Ferner kann der Koppler auch im Hauptteil 2 integriert sein.

Wie in Fig. 1 schematisch dargestellt ist, umfaßt die Abbildungsoptik 4 in der Richtung vom distalen Ende 5 bis zum proximalen Ende 8 hin ein Objektiv 12, ein erstes Optikmodul 13, drei hintereinander angeordnete Umkehrsysteme 14, ein zweites Optikmodul 15, ein Korrekturmodul 16 sowie ein Okular 17. Mittels des Objektives 12 wird das Objekt 6 aufgenommen und über das erste Optikmodul 13 in das erste Umkehrsystem 14 eingekoppelt, das in gleicher Weise wie die beiden weiteren Umkehrsysteme 14 ein eingangsseitig vorliegendes Bild jeweils so weiterleitet, daß es ausgangsseitig um 180° gedreht abgebildet vorliegt. Die drei Umkehrsysteme 14, die jeweils bevorzugt als Stablinsensystem ausgebildet sind, bilden somit ein Leitsystem, mit dem das Bild des aufgenommenen Objektes 6 zum Hauptteil 2 geleitet wird, in dem es mittels des zweiten Optikmoduls 15 dem Korrekturmodul 16 zugeführt wird und dann über das Okular 17 so abgebildet wird, daß es mittels der Kamera 9 aufgenommen werden kann. Die Abbildungsoptik 4 ohne das Korrekturmodul 16 kann beispielsweise wie in der deutschen Patentanmeldung Nr. 10 2005 032 515 A1 beschrieben ist, ausgebildet sein. Die beiden Optikmodule 13 und 15 sind optional und können gegebenenfalls auch weggelassen werden.

Das Korrekturmodul 16 dient, wie nachfolgend noch im Detail beschrieben wird, dazu, den chromatischen Längsfehler im Bereich der Fokusebene 7 zwischen Licht aus dem sichtbaren Spektrum und Licht aus dem nahen Infrarot zu kompensieren, da Endoskope immer häufiger dazu eingesetzt werden, neben einem Bild im sichtbaren Wellenlängenbereich auch noch ein Bild im nahen Infrarot aufzunehmen. So können beispielsweise Fluoreszenzstoffe in Krebsgewebe angereichert und zur Fluoreszenz bei 835 nm angeregt werden. Nachdem jedoch herkömmliche Abbildungsoptiken in der Regel nur für das sichtbare Spektrum korrigiert sind, liegt die Fokusebene für Licht aus dem nahen Infrarot nicht in der Fokusebene 7 sondern dahinter (in Fig. 1 rechts davon). Die Auslegung der Abbildungsoptik 4 derart, daß sie auch für Licht aus dem nahen Infrarot korrigiert ist, würde dazu führen, daß mehr optische Elemente notwendig sind, so daß die Herstellung aufwendiger und teurer werden würde. Darüber hinaus ist gerade im Bereich des Endoskopschaftes 3 in der Regel sehr wenig Platz für die Optik, so daß dies in nachteiliger Weise unter Umständen zu einem größeren Schaftdurchmesser führen würde.

Daher ist bei dem erfindungsgemäßen Endoskop 1 im Hauptteil das Korrekturmodul 16 angeordnet, das diesen chromatischen Längsfehler im Bereich der Fokusebene 7 korrigiert.

Dazu weist das Korrekturmodul 16, wie in der vergrößerten Detailansicht in Fig. 2 ersichtlich ist, einen Strahlteiler 18, ein erstes Umlenkprisma 19, eine Glasstrecke 20, ein zweites Umlenkprisma 21 sowie einen Strahlvereiniger 22 auf. Das vom zweiten Optikmodul 15 kommende Licht läuft in einem ersten Strahlengang 23 bis zum Strahlteiler 18, der Licht aus dem nahen Infrarot zum ersten Umlenkprisma 19 hin reflektiert und Licht aus dem sichtbaren Spektrum transmittiert. Das Licht aus dem nahen Infrarot wird vom ersten Umlenkprisma 19 umgelenkt, durchläuft die Glasstrecke 20 und wird dann vom zweiten Umlenkprisma 21 zum Strahlvereiniger 22 hin umgelenkt. Der Strahlvereiniger 22 reflektiert das Licht aus dem nahen Infrarot und transmittiert das Licht aus dem sichtbaren Spektrum, das vom Strahlteiler 18 entlang eines ersten Teilstrahlenganges 25 bis zum Strahlvereiniger 22 läuft, so daß der Strahlvereiniger 22 das Licht aus dem sichtbaren Spektrum und das Licht aus dem nahen Infrarot überlagert und das überlagerte Licht entlang eines zweiten Strahlenganges 24 zum Okular 17 führt.

Somit weist das Korrekturmodul 16 unterschiedliche Teilstrahlengänge 25, 26 auf, wobei der Teilstrahlengang für das Licht aus dem sichtbaren Spektrum als erster Teilstrahlengang 25 und der Teilstrahlengang für das Licht aus dem nahen Infrarot als zweiter Teilstrahlengang 26 bezeichnet wird. Das Korrekturmodul 16 ist nun so ausgelegt, daß die optischen Weglängen in den beiden Teilstrahlengängen 25 und 26 unterschiedlich gewählt sind. Der Unterschied in den optischen Weglängen ist so gewählt, daß damit der chromatische Längsfehler im Bereich der Fokusebene 7 (bevorzugt vollständig) kompensiert wird. Dies wird dadurch erreicht, daß die Materialien für die beiden Teilstrahlengänge 25 und 26 unterschiedlich gewählt werden. So ist beispielsweise im ersten Teilstrahlengang Luft mit einer Brechzahl n₀ von 1 für Licht aus dem sichtbaren Spektrum vorhanden. Der Strahlteiler 18 und der Strahlvereiniger 22 weisen jeweils eine Brechzahl n₁ auf, die größer ist als die Brechzahl n₀. So kann beispielsweise das Material N-BK10 für den Strahlteiler 18 und den Strahlvereiniger 22 verwendet werden, was eine Brechzahl für die Wellenlänge von 588 nm von 1,49782 und für eine Wellenlänge von 852 nm von 1,49127 aufweist.

Für die beiden Umlenkprismen 19 und 21 sowie die Glasstrecke 20 wird ein Material mit einer Brechzahl n₂ gewählt, die für Licht aus dem nahen Infrarot größer ist als die Brechzahl n₀ des ersten Teilstrahlengangs 25 für Licht aus dem sichtbaren Spektrum. Als Material kann beispielsweise S-LAH 79 mit einer Brechzahl von 1,97630 für eine Wellenlänge von 852 nm gewählt werden.

Über die Wahl der Länge des zweiten Teilstrahlenganges (also der Weg für das Licht aus dem nahen Infrarot im ersten Umlenkprisma 19, in der Glasstrecke 20 und im zweiten Umlenkprisma 21) unter Berücksichtigung der entsprechenden Brechzahlen n₀, n₁ und n₂ kann dann die gewünschte Kompensation des chromatischen Längsfehlers erfolgen. So kann im wesentlichen die Länge der Glasstrecke 20 variiert werden, um die gewünschte notwendige Korrektur zu erzielen.

Da der erste und zweite Strahlengang 23, 24 koaxial sind, kann der Platzbedarf des Korrekturmoduls 16 quer zur Längsrichtung der beiden Strahlengänge 23 und 24 minimiert werden. Für Licht aus dem sichtbaren Spektrum ist der erste Strahlengang 23, der erste Teilstrahlengang 25 sowie der zweite Strahlengang 24 ein sich geradlinig erstreckender Hauptstrahlengang und lediglich das Licht aus dem nahen Infrarot wird aus diesem Hauptstrahlengang ausgekoppelt, über den zweiten Teilstrahlengang 26 geführt und dann wieder in den Hauptstrahlengang eingekoppelt. Somit kann das Korrekturmodul 16 leicht so ausgelegt werden, daß die gewünschte Kompensation des chromatischen Längsfehlers im Bereich der Fokusebene 7 erzielt wird und gleichzeitig können die notwendigen räumlichen Abmessungen (insbesondere quer zur Längsrichtung) der Strahlengänge 23, 24 minimiert werden. Da das Korrekturmodul 16 im Hauptteil 2 des Endoskopes angeordnet ist, liegt genügend Platz für das Korrekturmodul 16 vor.

Durch die Ausbildung mit dem Strahlteiler 18 und dem Strahlvereiniger 22 sowie den beiden Umlenkprismen 19 und 21 und der Glasstrecke 20 können die einzelnen Elemente miteinander verkittet sein. Es liegt somit ein im wesentlichen U-förmiges Glasaufbau vor, der die gewünschte Korrektur des chromatischen Längsfehlers bewirkt.

In Fig. 3 ist eine Abwandlung des Korrekturmoduls 16 gezeigt. In diesem Fall ist der Strahlteiler 18 so ausgelegt, daß er Licht aus dem sichtbaren Spektrum reflektiert und Licht aus dem nahen Infrarot transmittiert. Ferner ist der Strahlvereiniger 22 so ausgelegt, daß er Licht aus dem nahen Infrarot transmittiert und Licht aus dem sichtbaren Spektrum reflektiert.

Auch hier wird der Vorteil erreicht, daß die beiden Strahlengänge 23 und 24 koaxial angeordnet sind. Auch liegt wiederum ein U-förmiger Glasaufbau vor.

Natürlich muß der Teilstrahlengang für das Licht aus dem sichtbaren Spektrum nicht durch Luft verlaufen. Man kann auch ein geeignetes Glasmaterial wählen. Wesentlich ist, daß die Brechzahl dieses Glasmaterials für Licht aus dem sichtbaren Spektrum kleiner ist als die Brechzahl der Glasstrecke 20 für Licht aus dem nahen Infrarot.

Das Korrekturmodul 16 ist als afokales Korrekturmodul 16 ausgebildet und weist keine gekrümmt ausgebildete Grenzfläche (insbesondere keine Linse) auf.

Natürlich müssen die Elemente des Korrekturmoduls 16 nicht aus Glas gebildet sein. Es ist auch jeder andere Werkstoff möglich. Beispielsweise kann Kunststoff verwendet werden.

## Patentansprüche

1. Endoskop mit
einem Hauptteil (2),
einem mit dem Hauptteil (2) verbundenen Endoskopschaft (3) und
einer innerhalb des Endoskopschaftes (3) und des Hauptteiles (2) angeordneten Abbildungsoptik (4), die ein vor dem dem Hauptteil (2) abgewandten Ende (5) des Endoskopschaftes (3) befindliches Objekt (6) in eine Fokusebene (7) der Abbildungsoptik (10) abbildet,
wobei das Hauptteil (2) an dem dem Endoskopschaft (3) abgewandten Ende (8) einen Kameraanschluss aufweist, an dem eine Kamera angebracht werden kann, wobei die Abbildungsoptik (4) ein Korrekturmodul (16) aufweist, durch das Licht zur Abbildung des Objektes (6) geführt ist und das eine Teilereinheit (18) und eine Überlagerungseinheit (22) umfaßt, wobei das Licht dem Korrekturmodul (16) über einen ersten Strahlengang (23) zugeführt ist, der mittels der Teilereinheit (18) in einen ersten und einen zweiten Teilstrahlengang (25, 26) aufgeteilt ist, wobei die Teilereinheit (18) Licht aus dem sichtbaren Spektrum in einen der beiden Teilstrahlengänge (25, 26) und Licht aus dem nahen Infrarot in den anderen der beiden Teilstrahlengänge (25, 26) führt,
und die Überlagerungseinheit (22) das Licht aus beiden Teilstrahlengängen (25, 26) überlagert und in einen zweiten Strahlengang (24) führt, der koaxial zum ersten Strahlengang (23) ist, wobei die optischen Weglängen der beiden Teilstrahlengänge (25, 26) für das Licht aus dem sichtbaren Spektrum und das Licht aus dem nahen Infrarot so verschieden gewählt sind, daß der chromatische Längsfehler im Bereich der Fokusebene (7) zwischen dem Licht aus dem sichtbaren Spektrum und dem Licht aus dem nahen Infrarot kompensiert ist,
**dadurch gekennzeichnet, dass** das Korrekturmodul (16) im Hauptteil (2) angeordnet ist und frei von abbildenden optischen Elementen ist.

2. Endoskop nach Anspruch 1, bei dem das Korrekturmodul (16) als afokales Korrekturmodul (16) ausgebildet ist.

3. Endoskop nach einem der obigen Ansprüche, bei dem der erste Teilstrahlengang (25) koaxial zu beiden Strahlengängen (23, 24) ist.

4. Endoskop nach einem der obigen Ansprüche, bei dem der zweite Teilstrahlengang (26) einen Abschnitt (20) aufweist, der parallel zu den beiden Strahlengängen (23, 24) ist.

5. Endoskop nach einem der obigen Ansprüche, bei dem der Teilstrahlengang (25, 26), in dem das Licht aus dem nahen Infrarot geführt ist, einen sich geradlinig erstreckenden Block (20) mit einer ersten Brechzahl, und der Teilstrahlengang (25, 26), in dem das Licht aus dem sichtbaren Spektrum geführt ist, ein Medium mit einer zweiten Brechzahl aufweist, wobei die erste Brechzahl für Licht aus dem nahen Infrarot größer ist als die zweite Brechzahl für Licht aus dem sichtbaren Spektrum.

6. Endoskop nach einem der obigen Ansprüche, bei dem der erste Teilstrahlengang (25) geradlinig und der zweite Teilstrahlengang (26) U-förmig ist.

7. Endoskop nach einem der obigen Ansprüche, bei dem die Teilereinheit (18) und die Überlagerungseinheit (22) jeweils als Teilerwürfel ausgebildet sind.

8. Endoskop nach Anspruch 7, bei dem der erste Teilstrahlengang (25) einen zwischen beiden Teilerwürfeln angeordneten, sich geradlinig erstreckenden Block aufweist, der in mechanischem Kontakt mit beiden Teilerwürfeln steht.

9. Endoskop nach einem der obigen Ansprüche, bei dem der zweite Teilstrahlengang (26) zwei Umlenkprismen (19, 21) aufweist.

10. Endoskop nach Anspruch 7 und 9, bei dem jedes Umlenkprisma (19, 21) mit jeweils einem der Teilerwürfel in mechanischem Kontakt steht.

11. Endoskop nach Anspruch 9 oder 10, bei dem der zweite Teilstrahlengang (26) einen sich geradlinig erstreckenden Block (20) aufweist, der zwischen den beiden Umlenkprismen (19, 21) angeordnet ist und mit diesen in mechanischem Kontakt steht.

## Claims

1. Endoscope comprising
a main part (2),
an endoscope shaft (3) connected to the main part (2), and an imaging optical unit (4) arranged within the endoscope shaft (3) and the main part (2), said imaging optical unit imaging an object (6) situated in front of the end (5) of the endoscope shaft (3) facing away from the main part (2) into a focal plane (7) of the imaging optical unit (10),
wherein the main part (2) comprises a camera connector at the end (8) facing away from the endoscope shaft (3), it being possible to attach a camera to said camera connector,
wherein the imaging optical unit (4) comprises a correction module (16), the light for imaging the object (6) being guided therethrough and a splitter unit (18) and a superposition unit (22) being comprised thereby, wherein the light is fed to the correction module (16) via a first beam path (23), which is split into a first and a second partial beam path (25, 26) by means of the splitter unit (18), wherein the splitter unit (18) guides light from the visible spectrum in one of the two partial beam paths (25, 26) and light from the near infrared and the other of the two partial beam paths (25, 26),
and the superposition unit (22) superposes the light from the two partial beam paths (25, 26) and guides said light in a second beam path (24) that is coaxial to the first beam path (23),
wherein the optical path lengths of the two partial beam paths (25, 26) for the light from the visible spectrum and the light from the near infrared are chosen to be so different that the longitudinal chromatic aberration between the light from the visible spectrum and the light from the near infrared is compensated in the region of the focal plane (7),
**characterized in that** the correction module (16) is arranged in the main part (2) and is free from imaging optical elements.

2. Endoscope according to Claim 1, wherein the correction module (16) is embodied as an afocal correction module (16).

3. Endoscope according to either of the preceding claims, wherein the first partial beam path (25) is coaxial to the two beam paths (23, 24).

4. Endoscope according to any one of the preceding claims, wherein the second partial beam path (26) has a portion (20) that is parallel to the two beam paths (23, 24) .

5. Endoscope according to any one of the preceding claims, wherein the partial beam path (25, 26) along which the light from the near infrared is guided comprises a block (20) with a first refractive index, said block extending in a straight line, and the partial beam path (25, 26) along which the light from the visible spectrum is guided comprises a medium with a second refractive index, wherein the first refractive index for light from the near infrared is greater than the second refractive index for light from the visible spectrum.

6. Endoscope according to any one of the preceding claims, wherein the first partial beam path (25) is straight-lined and the second partial beam path (26) is U-shaped.

7. Endoscope according to any one of the preceding claims, wherein the splitter unit (18) and the superposition unit (22) are each embodied as a splitter cube.

8. Endoscope according to Claim 7, wherein the first partial beam path (25) comprises a block that is arranged between the two splitter cubes and that extends in a straight line, said block being in mechanical contact with both splitter cubes.

9. Endoscope according to any one of the preceding claims, wherein the second partial beam path (26) comprises two deflection prisms (19, 21).

10. Endoscope according to Claim 7 and 9, wherein each deflection prism (19, 21) is in mechanical contact with one of the splitter cubes in each case.

11. Endoscope according to Claim 9 or 10, wherein the second partial beam path (26) comprises a block (20) that extends in a straight line, said block being arranged between the two deflection prisms (19, 21) and being in mechanical contact therewith.

## Revendications

1. Endoscope, comprenant
une partie principale (2),
une tige d'endoscope (3) reliée à la partie principale (2) et
une optique de représentation (4) disposée à l'intérieur de la tige d'endoscope (3) et de la partie principale (2), laquelle représente, dans un plan focal (7) de l'optique de représentation (10), un objet (6) qui se trouve devant l'extrémité (5) de la tige d'endoscope (3) à l'opposé de la partie principale (2),
la partie principale (2) possédant, à l'extrémité (8) à l'opposé de la tige d'endoscope (3), un raccord de caméra au niveau duquel peut être montée une caméra,
l'optique de représentation (4) possédant un module de correction (16) à travers lequel est guidée la lumière destinée à représenter l'objet (6) et qui comporte une unité de division (18) et une unité de superposition (22),
la lumière étant acheminée au module de correction (16) par le biais d'un premier trajet de faisceau (23), lequel est divisé au moyen de l'unité de division (18) en un premier et un deuxième trajet de faisceau partiel (25, 26), l'unité de division (18) guidant la lumière du spectre visible dans l'un des deux trajets de faisceau partiels (25, 26) et la lumière du proche infrarouge dans l'autre des deux trajets de faisceau partiels (25, 26), et l'unité de superposition (22) superposant la lumière issue des deux trajets de faisceau partiels (25, 26) et la guidant dans un deuxième trajet de faisceau (24), lequel est coaxial par rapport au premier trajet de faisceau (23),
les longueurs d'onde optiques des deux trajets de faisceau partiels (25, 26) pour la lumière du spectre visible et la lumière du proche infrarouge étant choisies différentes de sorte que l'erreur de longueur chromatique dans la zone du plan focal (7) entre la lumière du spectre visible et la lumière du proche infrarouge est compensée, **caractérisé en ce que** le module de correction (16) est disposé dans la partie principale (2) et est dépourvu d'éléments optiques de représentation.

2. Endoscope selon la revendication 1, avec lequel le module de correction (16) est réalisé sous la forme d'un module de correction (16) afocal.

3. Endoscope selon l'une des revendications précédentes, avec lequel le premier trajet de faisceau partiel (25) est coaxial par rapport aux deux trajets de faisceau (23, 24).

4. Endoscope selon l'une des revendications précédentes, avec lequel le deuxième trajet de faisceau partiel (26) possède une portion (20) qui est parallèle aux deux trajets de faisceau (23, 24).

5. Endoscope selon l'une des revendications précédentes, avec lequel le trajet de faisceau partiel (25, 26) dans lequel est guidée la lumière du proche infrarouge possède un bloc (20) qui s'étend de manière rectiligne et ayant un premier indice de réfraction, et le trajet de faisceau partiel (25, 26) dans lequel est guidée la lumière du spectre visible possède un milieu ayant un deuxième indice de réfraction, le premier indice de réfraction pour la lumière du proche infrarouge étant supérieur au deuxième indice de réfraction pour la lumière du spectre visible.

6. Endoscope selon l'une des revendications précédentes, avec lequel le premier trajet de faisceau partiel (25) est rectiligne et le deuxième trajet de faisceau partiel (26) est en forme de U.

7. Endoscope selon l'une des revendications précédentes, avec lequel l'unité de division (18) et l'unité de superposition (22) sont respectivement réalisées sous la forme de cubes diviseurs.

8. Endoscope selon la revendication 7, avec lequel le premier trajet de faisceau partiel (25) possède un bloc qui s'étend de manière rectiligne, disposé entre les deux cubes diviseurs et qui se trouve en contact mécanique avec les deux cubes diviseurs.

9. Endoscope selon l'une des revendications précédentes, avec lequel le deuxième trajet de faisceau partiel (26) possède deux prismes de déviation (19, 21).

10. Endoscope selon les revendications 7 et 9, avec lequel chaque prisme de déviation (19, 21) se trouve en contact mécanique respectivement avec l'un des cubes diviseurs.

11. Endoscope selon la revendication 9 ou 10, avec lequel le deuxième trajet de faisceau partiel (26) possède un bloc (20) qui s'étend de manière rectiligne, lequel est disposé entre les deux prismes de déviation (19, 21) et se trouve en contact mécanique avec ceux-ci.
